(19) Europäisches Patentamt
European Patent Office
Office européen des brevets

(11) Numéro de publication: **0 078 729**
**B1**

# (12) FASCICULE DE BREVET EUROPEEN

(45) Date de publication du fascicule du brevet: **08.01.86**

(51) Int. Cl.[4]: **C 07 C 119/048**, C 07 C 118/06

(21) Numéro de dépôt: **82401944.2**

(22) Date de dépôt: **22.10.82**

(54) **Procédé de fabrication d'isocyanates aromatiques par carbonylation des dérivés nitrés en présence de catalyseurs supportés préparés à partir de complexes hétéropolymétalliques.**

(30) Priorité: **03.11.81 FR 8120567**

(43) Date de publication de la demande:
**11.05.83 Bulletin 83/19**

(45) Mention de la délivrance du brevet:
**08.01.86 Bulletin 86/2**

(84) Etats contractants désignés:
**AT BE CH DE FR GB IT LI LU NL SE**

(56) Documents cités:
**FR - A - 2 452 318**

(73) Titulaire: **RHONE-POULENC CHIMIE DE BASE, 25, quai Paul Doumer, F-92408 Courbevoie (FR)**

(72) Inventeur: **Kervennal, Jacques, 134, rue Docteur Locard, F-69005 Lyon (FR)**
Inventeur: **Cognion, Jean-Marie, 85, route de Charly, F-69230 Saint-Genis Laval (FR)**
Inventeur: **Braunstein, Pierre, 13, rue du Faubourg de Saverne, F-67000 Strasbourg (FR)**

(74) Mandataire: **Varnière-Grange, Monique et al, RHONE-POULENC RECHERCHES Service Brevets Chimie et Polymères Centre de Recherches de Saint-Fons 85, avenue des Frères Perret B.P. 62, F-69192 St-Fons Cédex (FR)**

EP 0 078 729 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne un procédé de fabrication des isocyanates organiques à partir des composés nitrés et plus particulièrement un procédé de préparation en phase liquide des isocyanates aromatiques par réaction entre les dérivés nitrés aromatiques et le monoxyde de carbone en présence de catalyseurs hétérogènes constitués de particules métalliques ou d'oxydes métalliques déposés sur supports à partir de complexes moléculaires hétéropolymétalliques.

Les isocyanates aromatiques sont des intermédiaires organiques de grand intérêt. Deux d'entre eux sont particulièrement développés: le toluène diisocyanate et le diphénylméthane diisocyanate-4,4' qui sont utilisés dans la synthèse des polyuréthanes. Certains isocyanates aromatiques substitués sont également utilisés dans les synthèses d'herbicides. Les procédés industrialisés pour la préparation de ces produits font tous intervenir la réaction de phosgénation d'une amine provenant de l'hydrogénation catalytique d'un dérivé nitré. Les inconvénients de ces procédés sont multiples; ils nécessitent notamment la synthèse et la manipulation de phosgène, produit très dangereux, et ils sous produisent de l'acide chlorhydrique en quantités importantes ce qui exige l'implantation et l'entretien coûteux d'un atelier particulier pour l'électrolyse de cet acide afin de recycler le chlore.

L'intérêt que présente un procédé évitant l'utilisation du phosgène est évident. Dans plusieurs brevets sont décrites des compositions catalytiques permettant de préparer, à température et pression élevées, les isocyanates par réaction d'un composé organique nitré avec l'oxyde de carbone. Le rôle favorable des métaux nobles pour cette réaction est bien connu à partir de ces publications. C'est ainsi que dans le brevet français n° 1 600 529 est décrite l'utilisation comme catalyseur d'un halogénure de métal noble en présence d'une base aminée à caractère aromatique; le brevet allemand n° 1 910 303 décrit des catalyseurs formés d'halogénures ou d'oxydes de Ru, Rh, Pd, Os, Ir, Pt, et d'un composé soufré hétéroaromatique en présence éventuellement d'un oxyde de Cr, Mo, Nb, W, V; dans le brevet français n° 1 567 321 est décrite l'utilisation d'un système catalytique formé d'un halogénure de métal noble et d'un composé organique du phosphore, par exemple une triarylphosphine ou un phosphite. Dans le brevet français n° 2 155 242 sont décrites des systèmes catalytiques constitués d'un ou plusieurs halogénures de palladium et/ou de rhodium, d'une ou plusieurs bases azotées hétéroatomiques et d'un cocatalyseur formé d'un ou plusieurs borates de fer; dans le brevet français n° 2 120 110 la formulation catalytique comprend outre un halogénure de palladium et des bases hétérocycliques azotées, un cocatalyseur constitué d'un ou plusieurs molybdates de fer et/ou de manganèse.

Tous ces systèmes à base de métaux précieux permettent de produire des isocyanates à partir de composés nitrés avec des sélectivités et des productivités variables; cependant, leur récupération difficile et coûteuse empêche toute extrapolation industrielle.

Certaines tentatives on été faites pour déposer les phases actives sur des supports; c'est ainsi qu'il est envisagé dans les brevets français n° 1 600 529 et 1 558 986 de même que dans le brevet américain n° 3 728 370, sans illustration pratique, de déposer le catalyseur sur des supports du type alumine, silice, carbone, sulfate de baryum, carbonate de calcium, amiante, bentonite, terre à diatomées, terre à foulon. Le brevet anglais n° 1 257 932 décrit l'utilisation de palladium, chlorure de palladium ou chlorure de rhodium sur alumine, silice et carbure de silicium pour la carbonylation en phase vapeur des dérivés nitrés, et une publication de W.B. HARDY et R. P. BENNET dans Tetrahedron Letters n° 1, p. 961, (1967) donne les résultats obtenus avec un catalyseur au rhodium sur charbon additionné de $FeCl_3$ dans la carbonylation du nitrobenzène en phénylisocyanate.

Des catalyseurs supportés dont la phase active est constituée à la fois par du palladium et du molybdène sont connus; ainsi le brevet français n° 2 452 318 décrit la carbonylation de nitro-aromatiques à l'aide de catalyseurs constitués d'oxydes de palladium et de molybdène déposés par inprégnation successive de molybdate d'ammonium et de chlorure de palladium sur support alumine, silice ou silice-alumine.

Il a été découvert que l'utilisation comme précurseurs de la phase active de complexes moléculaires hétéropolymétalliques, c'est-à-dire de systèmes contenant des atomes métalliques de nature différente, conduit à des catalyseurs dont les performances dans la carbonylation des nitro-aromatiques sont très sensiblement améliorées. Ces complexes renferment un métal noble du groupe VIII et un deuxième métal situé dans les familles $V_B$, $VI_B$ ou VIII de la classification périodique. Les clusters mixtes bimétalliques sont de bons exemples, selon l'invention, de telles espèces moléculaires. Ces complexes sont constitués d'un coeur polymétallique dans lequel existent des intéractions directes entre les atomes des différents métaux, le rapport atomique des métaux pouvant varier d'une molécule à l'autre. Dans les clusters organométalliques, ce coeur est entouré de coordinats parmi lesquels se trouvent de façon non limitative le monoxyde de carbone, des phosphines aliphatiques ou aromatiques, des ligands organiques insaturés tels que les cyclopentadiényles.

Parmi les couples favorablement associés se trouvent les métaux suivants: palladium-molybdène, platine-molybdène, palladium-fer, platine-fer, palladium-tungstène, platine-tungstène.

Comme exemples non limitatifs de tels clusters, citons, le $Pd_2Mo_2(\eta^5\text{-}C_5H_5)_2\,(CO)_6\,(PEt_3)_2$ dont la synthèse a été décrite par R. BENDER; P. BRAUNSTEIN, Y. DUSAUSOY et J. PROTAS dans Angew. Chem. Int. Ed. Engl., 1978, <u>17</u>, 596. Le $Pt_2Mo_2\,(\eta^5\text{-}C_5H_5)_2\,(CO)_6\,(PEt_3)_2$ et le $Pt_2W_2\,(\eta^5\text{-}C_5H_5)_2\,(CO)_6\,(PPh_3)_2$ décrits par P. BRAUNSTEIN et coll. dans le Journal of Organometallic Chem. 1979, vol. 172, $C_{51}$.

Ils sont généralement solubles dans différents solvants organiques et on utilise préférentiellement des solvants chlorés tels que le chlorure de méthylène ou le chloroforme.

Les complexes moléculaires hétéropolymétalliques peuvent être déposés sur divers supports selon des techniques d'imprégnation pour conduire aux catalyseurs utilisés selon l'invention.

Ainsi le support, placé sous azote, peut être recouvert par exemple d'une solution du cluster dans un solvant organique tel que le chlorure de méthylène; l'ensemble est dégazé sous vide et le solvant distillé.

Une autre technique consiste à placer le support dans un évaporateur rotatif constamment et régulièrement agité et à l'asperger par exemple d'une solution du cluster dans un solvant organique, dans des conditions de température et de pression telles que celui-ci est évaporé au fur et à mesure de son introduction.

Le support imprégné est, après séchage, porté à au moins environ 300°C sous azote à l'aide d'une programmation linéaire de température à raison de 2°C/mn afin de décomposer le complexe métallique sous forme de particules de métal ou d'oxyde.

Les supports pouvant être utilisés sont divers; d'une façon non limitative on peut citer les alumines, silices, silice-alumines, oxyde de magnésium etc.

L'imprégnation peut être menée de façon à conduire à des teneurs en métaux sur le support 0,1 à 20% en poids et préférentiellement entre 1 et 15%.

Les composés nitrés sont mis en contact avec de l'oxyde de carbone à température et pression élevées en présence du catalyseur préparé selon le mode opératoire décrit ci-dessus. Il est possible d'opérer en présence d'un solvant organique, par une technique discontinue dans un appareillage du type autoclave ou par une technique continue qui permet d'éliminer dès sa formation l'isocyanate produit. L'équation de la réaction peut sécrire selon le schéma ci-cessous:

$$R-NO_2 + 3\,CO \longrightarrow R-NCO + 2\,CO_2$$

Le procédé selon l'invention est applicable aux composés aromatiques comportant un ou plusieurs groupements nitrés fixés sur un atome de carbone d'un noyau aromatique. Ces composés, connus comme matières de base à la préparation des mono et diisocyanates aromatiques, peuvent être représentés par la formule:

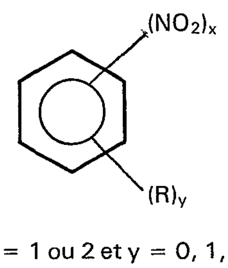

dans laquelle x = 1 ou 2 et y = 0, 1, 2, 3, R étant un groupement d'atomes ou un atome fixé sur le noyau aromatique et pouvant représenter un groupement alkyle ayant de 1 à 10 atomes de carbone, un atome d'halogène, chlore ou brome par exemple, ou un groupement alkoxy OR' dans lequel R' est un radical alkyle ayant de 1 à 10 atomes de carbone. Des exemples, non limitatifs, de composés aromatiques à une ou plusieurs fonctions nitrées, utilisables selon l'invention, sont le nitrobenzène, l'orthonitrotoluène, le paranitrotoluène, le dinitro-1,2 benzène, le dinitro-1,3 benzène, le dinitro-1,4 benzène, le dinitro-2,4 toluène, le dinitro-2,6 toluène, le méthoxy-1 dinitro-2,4 benzène, le chloro-1 nitro-2 benzène, le chloro-1 dinitro-2,4 nitro-2,4 benzène, le dichloro-3,4 nitrobenzène, le chloro-2 nitro-4 toluène, le chloro-3 bromo-4 nitrobenzène...

Le procédé selon l'invention est également applicable aux composés aromatiques nitrés possédant plusieurs noyaux substitués par une ou plusieurs fonctions nitrées et éventuellement par un ou plusieurs atomes d'halogène et/ou un ou plusieurs groupements alkyles, tels que, par exemple, de façon non limitative, le dinitrodiphénylméthane, le dinitro-2,4' stilbène, le dinitro-4,4' stilbène, le dinitro-2,4' bibenzyle, le dinitro-4,4' bibenzyle.

La concentration du catalyseur dans le milieu réactionnel, exprimée par le rapport du nombre d'atomes grammes du métal noble sur le nombre de groupements nitro à transformer peut varier entre $10^{-4}$ et 1 et préférentiellement entre $5.10^{3}$ et $10^{-1}$.

La réaction peut être conduite en l'absence de solvant, mais la présence d'un solvant favorise généralement la sélectivité en isocyanate. Les colvants utilisés de préférence sont les hydrocarbures saturés ou aromatiques tels que l'hexane, l'heptane, le n-décane, la décaline, le benzène, le toluène ou le xylène et des halogénures aromatiques tels que le chlorobenzène et les dichlorobenzènes; on peut également utiliser des solvants fluorés tels que la méthyldécaline perfluorée, le trichlorotrifluoroéthane.

Quand on opère en présence de solvant, la proportion n'est pas critique, mais on opère en général avec des solutions contenant de 5 à 50% en poids du dérivé nitré dans le solvant. Il est racommandé d'additionner au milieu réactionnel des quantités de pyridine, allant de 0,1 à 30 moles par mole de dérivé nitré et préférentiellement de 0,5 à 10 moles par mole de dérivé nitré en vue d'améliorer les sélectivités en isocyanate.

Les températures de réaction sont comprises entre 100°C et 500°C et plus particulièrement entre 150°C et 300°C suivant la nature et la stabilité des réactifs mis en jeu dans les conditions opératoires.

Les pressions de réaction sont comprises entre 20 et 500 bars, de préférence entre 150 et 350 bars, et doivent être suffisantes pour maintenir en phase liquide une fraction importante des réactifs et introduire une quantité totale d'oxyde de carbone correspondant à un rapport molaire $\frac{CO}{\text{groupement } NO_2}$ généralement compris entre 3 et 100 et de préférence entre 10 et 65.

Le procédé selon l'invention est particulièrement intéressant pour la fabrication du toluène diisocyanate et du phénylisocyanate qui, sous forme carbamatée avec un alcool léger tel que du méthanol ou de l'éthanol, peut servir de matière de base à la fabrication du diphénylméthane diisocyanate-4,4' selon la description faite dans Chemical Week, p. 57 du 9 novembre 1977.

Les essais décrits dans les exemples ci-dessous ont été réalisés en discontinu dans un autoclave en Hastelloy C de 500 ml, muni d'un dispositif d'agitation magnétique, pouvant opérer sous des pressions

allant jusqu'à 500 bars et des températures de 300°C. L'autoclave, chargé avec les différents réactifs, le solvant éventuel et le catalyseur, est ensuite balayé à l'azote avant d'être mis sous pression d'oxyde de carbone à température ordinaire. L'autoclave est chauffé à la température choisie et l'avancement de la réaction est contrôlé par enregistrement de la pression. Après réaction, les teneurs en isocyanates ont été évaluées par dosage chimique à la dibutylamine, et celles en dérivés nitrés résiduaires et éventuellement en dérivé azo par chromatographie en phase vapeur.

Après réaction, le catalyseur est facilement récupérable par filtration; il peut être recyclé tel quel ou subir avant réutilisation un traitement de régénération.

Les résultats indiqués s'entendent pour les définitions suivantes:

T.T.G., le taux de transformation globale =

$$\frac{\text{Nombre de moles de dérivé nitré transformé}}{\text{Nombre de moles de dérivé nitré introduit}} \times 100$$

Sélectivité en isocyanate =

$$\frac{\text{Nombre de moles d'isocyanate formé}}{\text{Nombre de moles de dérivé nitré transformé}} \times 100$$

Rendement en isocyanate =

$$\frac{\text{Nombre de moles d'isocyanate formé}}{\text{Nombre de moles de dérivé nitré introduit}} \times 100$$

*Exemple 1*

Dans un un ballon de 50 ml monté sur un évaporateur, on place 3,5 g d'une alumine pure commercialisée par CONDEA CHEMIE, de surface spécifique 250 $m^2/g$ et broyée en particules de 200 à 600 µm. On verse dans le ballon, préalablement balayé à l'azote, 20 ml d'une solution désoxygénée de 1 g du cluster mixte $Pd_2Mo_2(\eta^5\text{-}C_5H_5)_2(CO)_6(PPh_3)_2$ dans du chlorure de méthylène. Le cluster a été préparé selon le mode opératoire décrit par BRAUNSTEIN et coll. dans Angew. Chem. Int. Ed. Engl., 1978, 17, 596.

On distille le solvant sous pression réduite et, après séchage, transfère sous azote le support imprégné dans un tube de cuisson dont on élève progressivement la température à raison de 2°C/mn jusqu'à 300°C. L'analyse indique que le catalyseur ainsi préparé contient 2,5% de palladium et 3% de molybdène.

*Exemple 2*

On place dans l'autoclave de 500 ml en Hastelloy C précédemment décrit 1,8 g du catalyseur préparé suivant l'exemple 1, 10 g de nitrobenzène, 1 g de pyridine et complète à 100 ml le volume total à l'aide d'o.dichlorobenzène. Après balayage à l'azote, on comprime de l'oxyde de carbone jusqu'à une pression de 200 bars à 20°C. On isole l'autoclave et le chauffe, sous agitation à 240 °C pendant 70 minutes. On laisse refroidir et analyse le mélange. Le taux de transformation globale est de 100% et la sélectivité en phénylisocyanate de 71%.

*Exemple 3*

On introduit dans l'autoclave utilisé dans l'exemple 2, 10 g de nitrobenzène, 5 g de pyridine, 1,9 g du catalyseur préparé dans l'exemple 1 et complète le volume total à 100 ml à l'aide d'orthodichlorobenzène. Après balayage du réacteur par de l'azote, on place l'autoclave sous une pression de 200 bars de monoxyde de carbone, puis, après l'avoir isolé, élève la température à 220°C tout en agitant le mélange. Après 4 heures de réaction, on coupe le chauffage et analyse le contenu de réacteur. Le T.T.G. du nitrobenzène est de 100% et la sélectivité en phénylisocyanate de 80%.

*Exemple 4*

On place dans un ballon à solides, monté sur un évaporateur rotatif, 3,5 g d'alumine identique à celle utilisée dans l'exemple 1, et broyée en particules de 200 à 600 µm. On laisse dégazer le support pendant 15 minutes sous une pression partielle de 100 mm de mercure. On introduit ensuite goutte à goutte une solution désoxygénée de 1 g de cluster $Pd_2Mo_2(\eta^5\text{-}C_5H_5)_2(CO)_6(PPh_3)_2$ dans 20 ml de chlorure de méthylène en ajustant la température du bain d'huile de l'évaporateur de façon que la distillation du solvant soit instantanée. Après introduction de la totalité de la solution, le support imprégné et séché est transféré sous azote dans un réacteur tubulaire pour y subir un traitement thermique analogue à celui décrit dans l'exemple 1. L'analyse indique que les teneurs respectives en palladium et molybdène du catalyseur obtenu sont de 2,5% et 2,7%.

*Exemple 5*

On introduit dans l'autoclave utilisé dans l'exemple 2, 10 g de nitrobenzène, 1 g de pyridine, 1,8 g du catalyseur préparé à l'exemple 4 et ajuste le volume total à 100 ml avec de l'orthodichlorobenzène. Après balayage à l'azote, on comprime de l'oxyde de carbone jusqu'à une pression de 200 bars puis isole le réacteur et le chauffe à 240°C sous agitation pendant 70 minutes. On laisse refroidir et analyse la mélange. Le taux de transformation globale est de 100% et la sélectivité en phénylisocyanate de 71,5%.

*Exemple 6* (comparatif)

On place dans un ballon monté sur un évaporateur rotatif, 3,7 g d'alumine identique à celle utilisée dans l'exemple 1 et broyée en grains de 200 à 600 µm. On verse dans le ballon, préalablemment balayé à l'azote 60 ml d'une solution aqueuse ammoniacale contenant 0,42 g d'acétate de palladium et 0,18 g de molybdate d'ammonium. On distille l'eau sous pression réduite et, après séchage, place le support imprégné dans un tube de cuisson pour y subir un traitement thermique analogue à celui décrit dans l'exemple 1. L'analyse indique que le catalyseur ainsi préparé contient 3,4% de palladium et 1,7% de molybdène.

On charge dans l'autoclave utilisé à l'exemple 2, 1,8 g de ce catalyseur ainsi que 10 g de nitrobenzène et 1 g de pyridine et complète le volume total à 100 ml à l'aide d'o.dichlorobenzène. En opérant de façon identique à l'exemple 2 à 240°C sous 200 bars de

monoxyde de carbone à 20°C, on obtient un T.T.G. du nitrobenzène de 100% et une sélectivité en phénylisocyanate de 62%.

*Exemple 7*

En opérant de la même façon que dans l'exemple 1, on imprègne 4 g de billes d'alumine commerciales de granulométrie comprise entre 200 et 600 µm et de surface spécifique 350 $m^2/g$, à l'aide d'une solution de 1,07 g de cluster mixte $Pd_2Cr_2$ ($\eta^5$-$C_5H_5)_2$ $(CO)_6$ $(PPh_3)_2$ dans du chlorure de méthylène. Après traitement thermique à 350°C, l'analyse indique que le catalyseur ainsi préparé contient 4% de palladium et 1,9% de chrome.

On place dans l'autoclave 10 g de nitrobenzène, 2,1 g du catalyseur, 1 g de pyridine et complète à 100 ml de volume total à l'aide d'orthodichlorobenzène. Après introduction de 200 bars de monoxyde de carbone, on chauffe le réacteur à 200°C sous agitation pendant 3 heures puis à 240°C pendant 1 H 20. On laisse refroidir et analyse le mélange. Le T.T.G. du nitrobenzène est de 90,8% et la sélectivité en phénylisocyanate de 65%.

*Exemple 8*

Suivant le mode opératoire décrit dans l'exemple 1, on imprègne 3 g de l'alumine utilisée dans l'exemple 7 avec une solution de 1 g de cluster mixte $Pd_2W_2$ ($\eta_5$-$C_5H_5)_2$ $(CO)_6$ $(PPh_3)_2$ dans du chlorure de méthylène. Après traitement thermique à 350°C, les dosages effectués sur le catalyseur indiquent des teneurs respectives de 3,6% et 6,2% en palladium et tungstène.

10 g de nitrobenzène, 2,1 g du catalyseur et 1 g de pyridine sont itroduits dans l'autoclave et le volume complété à 100 ml à l'aide d'orthodichlorobenzène. On introduit 200 bars de monoxyde de carbone et chauffe, sous agitation, le réacteur à 240°C. Après 1 H 20, on laisse refroidir et analyse le mélange. Le T.T.G. du nitrobenzène est de 100% et la sélectivité en phénylisocyanate de 56%.

*Exemple 9*

En opérant suivant le mode opératoire décrit dans l'exemple 1, on imprègne 4 g de support alumine identique à celui utilisé dans l'exemple 7 à l'aide d'une solution de 0,8 g de cluster mixte $Pt_2W_2$ ($\eta^5$-$C_5H_5)_2$ $(CO)_6$ $(PPh_3)_2$ dans du chlorure de méthylène. Après traitement thermique à 350°C l'analyse indique que le catalyseur contient 4% de platine et 3,7% de tungstène.

On introduit dans l'autoclave 10 g de nitrobenzène, 3 g de catalyseur, 1 g de pyridine et complète à 100 ml le volume total à l'aide d'orthodichlorobenzène. Après introduction de 200 bars de monoxyde de carbone, on chauffe le réacteur à 240°C, sous agitation, pendant 3 H 15 puis laisse refroidir. Le mélange est analysé, ce qui indique que le T.T.G. du nitrobenzène est de 96% et la sélectivité en phénylisocyanate de 57%.

**Revendications**

1. Procédé de fabrication d'isocyanates aromatiques par carbonylation de dérivés nitrés en présence de catalyseur contenant comme phase active, déposés sur un support, des particules ou un oxyde d'un métal noble du groupe VIII et d'un métal choisi dans les familles $V_B$, $VI_B$ et VIII de la classification périodique, caractérisé en ce que le précurseur de la phase active est un complexe moléculaire hétéropolymétallique.

2. Procédé selon la revendication 1, caractérisé en ce que le complexe hétéropolymétallique est un cluster mixte bimétallique.

3. Procédé selon l'une des revendications 1 à 2, caractérisé en ce que l'on imprègne un support d'une solution de complexe hétéropolymétallique et que l'on fait subir à l'ensemble un traitement thermique préalablement à l'utilisation du catalyseur.

4. Procédé selon l'une des revendications 1 à 3, caractérisé en ce que la concentration en poids des métaux sur le support est compris entre 0,1 et 20% du poids de l'ensemble du système catalytique.

5. Procédé selon l'une des revendications 1 à 4, caractérisé en ce que le catalyseur est utilisé dans un milieu réactionnel contenant de la pyridine comme solvant.

6. Procédé selon l'une des revendications 1 à 5, caractérisé en ce que le catalyseur se trouve dans un milieu réactionnel dont la pression de CO à 20°C est comprise entre 20 et 500 bars.

7. Procédé selon l'une des revendications 1 à 6, caractérisé en ce que le catalyseur se trouve dans un milieu réactionnel dont la température est comprise entre 100 et 500°C.

**Patentansprüche**

1. Verfahren zur Herstellung von aromatischen Isocyanaten durch Carbonylieren von Nitroverbindungen in Gegenwart von Katalysatoren, die als aktive, auf einem Träger abgeschiedene Phase Teilchen oder ein Oxid eines Edelmetalls der Gruppe VIII und eines Metalls aus den Gruppen $V_B$, $VI_B$ und VIII des Periodensystems enthalten, dadurch gekennzeichnet, dass der Vorläufer der aktiven Phase ein Heteropolymetall-Molekülkomplex ist.

2. Verfahren nach Anspruch 1, dadurch gekennzeichnet, dass der Heteropolymetallkomplex ein gemischter Bimetall-cluster ist.

3. Verfahren nach Anspruch 1 oder 2, dadurch gekennzeichnet, dass man einen Träger mit einer Lösung des Heteropolymetallkomplexes immprägniert und das Ganze vor der Verwendung des Katalysators einer Wärmebehandlung unterwirft.

4. Verfahren nach einem der Ansprüche 1 bis 3, dadurch gekennzeichnet, dass die auf das Gewicht bezogene Konzentration der Metalle auf dem Träger 0,1 bis 20 Gew.-% des gesamten Katalysatorsystems ausmacht.

5. Verfahren nach einem der Ansprüche 1 bis 4, dadurch gekennzeichnet, dass der Katalysator in einem Reaktionsmedium verwendet wird, das als Lösungsmittel Pyridin enthält.

6. Verfahren nach einem der Ansprüche 1 bis 5, dadurch gekennzeichnet, dass der Katalysator in einem Reaktionsmedium vorhanden ist, dessen CO-Druck bei 20°C 20 bis 500 bar beträgt.

7. Verfahren nach einem der Ansprüche 1 bis 6, dadurch gekennzeichnet, dass der Katalysator in einem Reaktionsmedium vorhanden ist, dessen Temperatur 100 bis 500°C beträgt.

**Claims**

1. Process for the manufacture of aromatic isocyanates by carbonylation of nitro derivatives in the presence of catalysts containing as an active phase, deposited on a support, particles or an oxide of a noble metal of group VIII and of a metal chosen from groups $V_B$, $VI_B$ and VIII of the periodic table, characterised in that the precursor of the active phase is a heteropolymetallic molecular complex.

2. Process according to Claim 1, characterised in that the heteropolymetallic complex is a mixed bimetallic cluster.

3. Process according to either of Claims 1 and 2, characterised in that a support is impregnated with a solution of a heteropolymetallic complex and that the whole is subjected to a heat treatment before the use of the catalyst.

4. Process according to one of Claims 1 to 3, characterised in that the weight concentration of the metals on the support is between 0.1 to 20% of the weight of the whole of the catalyst system.

5. Process according to one of Claims 1 to 4, characterised in that the catalyst is employed in a reaction medium containing pyridine as a solvent.

6. Process according to one of Claims 1 to 5, characterised in that the catalyst is in a reaction medium in which the pressure of CO at 20°C is between 20 and 500 bars.

7. Process according to one of Claims 1 to 6, characterised in that the catalyst is in a reaction medium the temperature of which is between 100 and 500°C.